# EUROPEAN PATENT APPLICATION

(11) **EP 4 378 497 A1**
(43) Date of publication of application: **05.06.2024**
(21) Application number: 22849621.2
(22) Date of filing: 29.07.2022
(51) Int. Cl.: A61M 1/36

(54) **BLOOD COMPONENT ADSORBENT MATERIAL**

(30) Priority: 30.07.2021 JP 2021125206
(71) Applicant: Toray Industries, Inc., Tokyo 103-8666 (JP)
(72) Inventor: KANDA, Shungo, Otsu-shi, Shiga 520-8558 (JP); YAMASHITA, Kyohei, Otsu-shi, Shiga 520-8558 (JP); KOMACHI, Shunsuke, Otsu-shi, Shiga 520-8558 (JP); TAKAHASHI, Hiroshi, Otsu-shi, Shiga 520-8558 (JP)
(74) Representative: Kador & Partner Part mbB
(86) International application number: PCT/JP2022/029308
(87) International publication number: WO 2023/008561

(57) **Abstract**

[Problem] To provide a blood component adsorbent material capable of adsorbing a blood component such as various leucocytes without limitation to the kind of a monocyte or a granulocyte.

[Solution] The present invention provides a blood component adsorbent material including: porous particles composed only of a first polymer having an aromatic hydrocarbon group in a repeating unit; and a second polymer having a hydroxy group in a repeating unit, and fixed to the surfaces of the porous particles, wherein the ratio of content of the second polymer in the surface of the blood component adsorbent material is 3 to 30 mg/g.

## Description

### Technical Field

The present invention relates to a blood component adsorbent material.

### Background Art

In recent years, a blood processing technology for removing cytokines or leucocytes, particularly a material for removing leucocytes from blood has been developed for the purpose of immunodepression before and after the treatment or transplantation of an inflammatory disease and for the purpose of inhibiting a side effect of a blood preparation, such as fever or infection. One of such technologies being developed is a technology for adsorptively removing leucocytes from blood by passing blood through a column including: a material the surface of which contains a ligand or resin having affinity with leucocytes; or a material having a given surface roughness and thus being more capable of adsorbing leucocytes.

In particular, a blood component adsorbent material in particulate form has a smaller specific surface area, is less prone to be clogged by filtration, and is suitable for blood processing. Thus, various adsorbent materials in particulate form are being developed.

For example, Patent Literature 1 discloses a blood component adsorbent carrier for adsorptive removal of both cytokines and leucocytes, the carrier including a water-insoluble carrier, wherein, to a functional group in the surface of the water-insoluble carrier, a sugar chain selected from the group consisting of sucrose, lactose, maltose, trehalose, and cellobiose is covalently bound, and wherein the sugar chain is covalently bound to an amino group of the functional group at the reducing terminal side.

In addition, Patent Literature 2 discloses a blood component adsorbent carrier characterized by including: at least an outer layer composed of a molded product of cellulose acetate; and an inner layer composed of polycarbonate and formed inside the outer layer.

Patent Literature 3 discloses a polymer system including at least one polymer, wherein the polymer contains a residue(s) of one or more aromatic monomers and one or more cross-linking agents, wherein the polymer has an external surface and a plurality of pores, and wherein the polymer is functionalized by different functional groups on the external surface and the surfaces in the pores.

Patent Literature 4 discloses coated particles including hydrophobic porous polymer particles and a coating layer composed of a cross-linked hydroxy group-containing polymer, and coating at least part of the surface of the hydrophobic porous polymer particle.

Patent Literature 5 discloses a biocompatible polymer system including at least one polymer, wherein the polymer contains one of a polyol and a zwitterionic functional group, wherein the polymer contains a solid support having a biocompatible hydrogel coating containing a hydroxy group in a repeating unit thereof, and wherein the polymer system can adsorb one or more of Gram-positive bacteria, a Gram-positive bacteria fragment, and a Gram-positive bacteria component.

In addition, Non-Patent Literature 1 discloses that an alveolar macrophage which is one kind of leucocyte specifically recognizes and adheres to a specific sugar chain.

### Citation List

### Patent Literature

Patent Literature 1: JP5644149B2
Patent Literature 2: JP3513155B2
Patent Literature 3: JP5913368B2
Patent Literature 4: JP2021-7915A
Patent Literature 5: JP7033083B2

### Non-Patent Literature

Non-Patent Literature 1: Largent et al., J. Biol. Chem., 1984, Vol. 259, pp. 1764-1769

### Summary of Invention

### Technical Problem

However, a conventional blood component adsorbent material in particulate form, such as for leucocytes, involves specific removal based on hydrogen bonding due to the sugar-derived molecular structure, and hence, as disclosed in Non-Patent Literature 1, has a problem in that the material can adsorb only some kinds of blood corpuscles among the leucocytes, such as an alveolar macrophage that specifically interacts with a sugar chain.

Leucocytes are classified into monocytes, granulocytes, and lymphocytes, and, in the adsorption of a blood component, monocytes and granulocytes are involved as components that initiate inflammation. Thus, it is considered preferable that monocytes and granulocytes are adsorbed without being particularly distinguished.

Patent Literature 1 and Patent Literature 2 disclose a material that adsorbs a blood component on the basis of bonding due to a sugar-derived molecular structure. Patent Literature 1 discloses that the material therein can adsorb neutrophils, which are one kind of granulocyte, and monocytes, but the Literature neither suggests nor describes whether the material can adsorb all of the monocytes and granulocytes. Furthermore, Patent Literature 1 describes the incorporation of a sugar chain for specific bonding of leucocytes, but neither suggests nor describes adsorptive removal based on a van der Waals force due to the physical structure of the surface of the material. In addition, Patent Literature 2 describes no specific example of a blood component that can be adsorbed.

Patent Literature 3 describes a material that can adsorptively remove a protein, toxin, and pathogen, but discloses no specific example of the effect at all, and neither discloses nor suggests the adsorptive removal of a cell component such as a leucocyte.

Patent Literature 4 describes a separating material for chromatography, and is based on the assumption that the material is used for the purpose of reversible adsorption of a protein. The material cannot be directly used for irreversible adsorptive removal of a blood component for the purpose of treating a patient. In addition, the Literature does not at all describe adsorptive removal of a cell component such as a leucocyte.

Patent Literature 5 discloses a biocompatible polymer system in which, for example, a polyol functional group, such as a diol group, or a zwitterionic functional group is provided on a solid support to remove one kind of bacteria-derived component, and in which a hydrogel coating is further provided to prevent the adsorption of a blood component into the surface of the solid support. However, the Literature neither suggests nor describes such adsorptive removal of a blood component as is based on a van der Waals force due to the physical structure. In addition, the Literature does not at all describe adsorptive removal of a cell component such as a leucocyte.

As above-described, no development of a material has hitherto been achieved, in which, utilizing a van der Waals force due to the physical structure of the surface of a material, the material is capable of adsorbing a blood component such as a leucocyte in a wide range without limitation to the kind of a monocyte and a granulocyte.

In view of this, an object of the present invention is to provide a blood component adsorbent material capable of adsorbing a blood component such as various leucocytes without limitation to the kind of a monocyte or a granulocyte, in which the adsorption is based on fixing a specific content of a polymer having a hydroxy group to the surfaces of porous particles composed only of a polymer having an aromatic hydrocarbon group, and thus controlling a van der Waals force on the surface of the blood component adsorbent material.

### Solution to Problem

The present inventors have advanced studies vigorously to solve the above-described problems, and consequently discovered the inventions according to the following (1) to (6).
(1) A blood component adsorbent material including: porous particles composed only of a first polymer having an aromatic hydrocarbon group in a repeating unit; and a second polymer having a hydroxy group in a repeating unit, and fixed to the surfaces of the porous particles, wherein the ratio of content of the second polymer in the surface of the blood component adsorbent material is 3 to 30 mg/g.
(2) The blood component adsorbent material according to (1), wherein the ratio of content of the second polymer in the blood component adsorbent material as a uniformized whole is 0.3 mg/g or less, and wherein the volume of the pores is 0.5 to 1.8 cm³ per 1 g of the material.
(3) The blood component adsorbent material according to (1) or (2), wherein the first polymer is polystyrene, polyethylvinylbenzene, polydivinylbenzene, poly(ethylvinylbenzene/divinylbenzene), or poly(styrene/divinylbenzene).
(4) The blood component adsorbent material according to any one of (1) to (3), wherein the second polymer is a polymer selected from the group consisting of polyhydroxyalkyl acrylate, polyhydroxyalkyl methacrylate, and polyvinyl alcohol.
(5) The blood component adsorbent material according to any one of (1) to (4), for use in the adsorptive removal of leucocytes.
(6) A blood purification column including the blood component adsorbent material according to any one of (1) to (5).

### Advantageous Effects of Invention

A blood component adsorbent material according to the present invention can adsorb a blood component such as various leucocytes with high efficiency without limitation to the kind of a monocyte or a granulocyte, in which the adsorption is based on a van der Waals force due to the physical structure of the surface.

### Description of Embodiments

Below, the present invention will be described in detail.

A blood component adsorbent material according to the present invention is characterized by including: porous particles composed only of a first polymer having an aromatic hydrocarbon group in a repeating unit; and a second polymer having a hydroxy group in a repeating unit, and fixed to the surfaces of the porous particles, wherein the ratio of content of the second polymer in the surface of the blood component adsorbent material is 3 to 30 mg/g.

The "first polymer having an aromatic hydrocarbon group in a repeating unit" means a polymer a repeating unit of which has a hydrocarbon group containing an aromatic structure. The polymer is not particularly limited as long as the repeating unit contains an aromatic hydrocarbon group. The polymer may contain a plurality of repeating units. The aromatic hydrocarbon group is preferably, for example, a functional group having a hydrocarbon structure substituted with any one of a benzene ring, naphthalene ring, and acetylene ring, and is more preferably contains a benzene ring.

The first polymer having an aromatic hydrocarbon group in a repeating unit may be a homopolymer composed of a single kind of repeating unit or a copolymer containing a plurality of kinds of repeating units, and in either case, preferably contains divinylbenzene from the viewpoints of preventing the dissolution of particles, and securing the strength.

In a case where the first polymer having an aromatic hydrocarbon group in a repeating unit is a homopolymer, the first polymer is preferably polystyrene, polyethylvinylbenzene, polymethylvinylbenzene, or polydivinylbenzene from the viewpoint of, for example, easiness of handling and availability, and, is preferably polydivinylbenzene particularly from the viewpoint of securing the strength of the particles. In this regard, the substituent on each aromatic ring is regardless of the position of substitution.

The composition of the first polymer having an aromatic hydrocarbon group in a repeating unit is not limited to any composition other than the composition containing a repeating unit composed of an aromatic hydrocarbon group, and may be a copolymer containing a plurality of kinds of repeating units. From the viewpoint of securing the strength, the copolymer is preferably, for example, poly(ethylvinylbenzene/divinylbenzene), poly(methylvinylbenzene/divinylbenzene), poly(styrene/divinylbenzene), or the like, which contains divinylbenzene. The copolymer is more preferably poly(ethylvinylbenzene/divinylbenzene).

The repeating number and molecular weight of the chemical structure of the first polymer having an aromatic hydrocarbon group in a repeating unit are not particularly limited. From the viewpoints of properties and strength stability of the polymer, the polymer preferably has 100 or more repeating units linked by a covalent bond.

The fact that the first polymer having an aromatic hydrocarbon group is a polymer having 100 or more covalently bound repeating units can be verified by the fact that the polymer does not dissolve when impregnated with isopropyl alcohol.

The first polymer having an aromatic hydrocarbon group in a repeating unit may have a cross-linked structure for retaining the strength and an initiator-derived terminal structure for the start and stop of polymerization.

The fact that the first polymer having an aromatic hydrocarbon group has a cross-linked structure can be verified by the fact that the first polymer does not dissolve when impregnated with acetone, and refluxed in a soxhlet extraction device.

The "second polymer having a hydroxy group in a repeating unit" means a polymer the chemical structure of which contains a hydroxy group in a repeating unit. The molecular structure and molecular weight of the repeating unit are not limited, and the molecular weight of the repeating unit is preferably 200 g/mol or less per hydroxy group.

The second polymer having a hydroxy group in a repeating unit is preferably, for example, a synthetic polymer such as polyvinyl alcohol, polyhydroxyalkyl acrylate, or polyhydroxy acrylate, or a polysaccharide such as dextran, dextran sulfate, alginic acid, hyaluronic acid, pectin, xyloglucan, xylan, or heparin, more preferably a synthetic polymer from the viewpoints of sterilizing ability and stability, still more preferably polyvinyl alcohol, polyhydroxyalkyl acrylate, or polyhydroxy acrylate.

From the viewpoint of easiness of fixation to porous particles, the second polymer having a hydroxy group in a repeating unit may be a copolymer containing a repeating unit constituted only by a hydrocarbon. Preferable examples of the repeating unit constituted only by a hydrocarbon include a repeating unit of polyethylene, polypropylene, polystyrene, polyethylvinylbenzene, polymethylvinylbenzene, polydivinylbenzene, or the like. Preferable examples of the second polymer include poly(vinyl alcohol/ethylene), poly(vinyl alcohol/styrene), poly(vinyl alcohol/methylvinylbenzene), poly(vinyl alcohol/divinylbenzene), poly(hydroxyalkyl acrylate/ethylene), poly(hydroxyalkyl acrylate/styrene), poly(hydroxyalkyl acrylate/methylvinylbenzene), poly(hydroxyalkyl acrylate/divinylbenzene), poly(hydroxy acrylate/ethylene), poly(hydroxy acrylate/styrene), poly(hydroxy acrylate/methylvinylbenzene), and poly(hydroxy acrylate/divinylbenzene).

The abundance ratio of a repeating unit containing a hydroxy group in a repeating unit of the second polymer is subject to no prescription, but the ratio, if too small, increases the hydrophobicity to thereby increase the ratio of adhesion of platelets, thus leading to clogging the column, and hence, is preferably 10% or more and 100% or less, more preferably 40% or more and 100% or less, particularly preferably 95% or more and 100% or less.

The repeating number (hereinafter referred to as a degree of polymerization) and molecular weight of the chemical structure of the second polymer having a hydroxy group in a repeating unit is not particularly limited. From the viewpoints of the interaction of the surface and modifiability, the polymer is preferably a polymer having 10 or more and 10000 or less covalently bound repeating units, and having a degree of polymerization of 10 to 10000, more preferably a degree of polymerization of 30 to 10000.

In a case where a commercially available polymer having a prescribed degree of polymerization is directly used, the degree of polymerization is adopted as the degree of polymerization of the second polymer. In a case where the degree of polymerization is not prescribed, the degree of polymerization is obtained by dividing the weight-average molecular weight by the molecular weight of the repeating unit, and rounding the units digit. For example, in a case where the weight-average molecular weight is 10000, and where the molecular weight of the repeating unit is 250, the degree of polymerization is 40. In a case where the second polymer is a copolymer composed of two or more kinds of monomers, a weighted average obtained by multiplying the abundance ratio of each repeating unit by the molecular weight of the repeating unit, and dividing the total of the resulting values by the number of all the components is regarded as the molecular weight of the repeating unit.

The second polymer having a hydroxy group in a repeating unit may have a cross-linked structure for retaining the strength and an initiator-derived terminal structure for the start and stop of polymerization.

The "porous particle" means a particle having a plurality of pores, and a porous particle in the present invention is constituted only by the first polymer.

The volume of the pores contained in the blood component adsorbent material is not limited. To enhance the strength of the blood component adsorbent material to further stabilize the adsorption treatment, the volume of the pores per 1 g of the blood component adsorbent material is preferably 0.5 cm³ or more and 1.8 cm³ or less.

The surface of the blood component adsorbent material means the properties along a given depth in the blood component adsorbent material, in which the properties are measured on an infrared absorption spectrum (at a wavenumber of 4500 cm⁻¹ to 500 cm⁻¹) by an attenuated total reflection (ATR) measurement method. The infrared absorption spectrum of the surface of the above-described blood component adsorbent material can be measured by the following method. On a Nicolet iS5 FT-IR (manufactured by Thermo Scientific; with iD5 diamond ATR accessories; the detector, DTGS KBr; the beam splitter, KBr), the Advanced ATR mode is selected, and the parameters (the number of scans, 16; the data interval, 0.241 cm⁻¹; without automated atmospheric correction) are set. After the setting, a background measurement is made. When the background measurement has ended, 1 g of the blood component adsorbent material preliminarily dried with a warm air drier at 60°C for 4 hours is spread over a prism, and pressed against the prism until the pressure device is locked. A measurement is started within 20 minutes after the background measurement, and then, an infrared absorption spectrum of the surface of the blood component adsorbent material can be obtained. In this regard, the range of measurement is set at a wavenumber of 4500 cm⁻¹ to 500 cm⁻¹.

The blood component adsorbent material as a uniformized whole means the blood component adsorbent material having the properties measured on an infrared absorption spectrum (at a wavenumber of 4500 cm⁻¹ to 500 cm⁻¹) by an attenuated total reflection (ATR) measurement method after the structures in the blood component adsorbent material are uniformized. The infrared absorption spectrum of the blood component adsorbent material as a uniformized whole can be analyzed by the following method. In a mortar (an agate mortar manufactured by As One Corporation; the deep type; 70 in diameter × 90 in diameter × 30 mm), 1 g of the blood component adsorbent material preliminarily dried with a warm air drier at 60°C for 4 hours is placed and ground down using a pestle 100 times with a force that at least cracks the blood component adsorbent material. The whole blood component adsorbent material is ground down one or more times, and the uniformization of the structure of the blood component adsorbent material is verified by visual observation. Thus, powder of the blood component adsorbent material as a uniformized whole is obtained. Except that the resulting powder is used in place of the blood component adsorbent material, a measurement is made by the same method as the measurement of an infrared absorption spectrum of the surface of the blood component adsorbent material, thus making it possible to obtain an infrared absorption spectrum of the blood component adsorbent material as a uniformized whole.

That the second polymer is fixed to the surfaces of the porous particles means that the second polymer fixed physically or chemically to the surfaces of the porous particles composed of the first polymer. The manner of the physical fixation is not limited, and is preferably fixation due to the aggregation on the surfaces of the porous particles or fixation due to the entanglement of the polymer. In addition, the manner of the chemical fixation is not limited, and is preferably fixation in the form of covalent bonding or ionic bonding.

The method of fixation of the second polymer to the surfaces of the porous particles may be physical or chemical fixation, and chemical fixation is more preferable from the viewpoints of being less prone to generate an eluate and being stable in a long-time storage.

The method of chemically fixing the second polymer is not particularly limited. From the viewpoint of stability, the second polymer is preferably fixed by covalent bonding, and preferably fixed via a linker.

A "linker" is a chemical structure for fixing the first polymer and the second polymer by covalent bonding, and means a chemical structure formed by covalent bonding between a repeating unit of the first polymer and a repeating unit of the second polymer. For example, in a case where a substituent is incorporated in an aromatic ring of the first polymer, and allowed to react with a hydroxy group of the second polymer to form a bond, the linker means a structure from a carbon of the aromatic ring to an oxygen atom of the hydroxy group after the reaction. The chemical structure contained as a linker is not limited, and is preferably, for example, an electrically neutral chemical bond such as an amide bond, alkylene group, urea bond, ether bond, or ester bond from the viewpoint of being bound to the first polymer that is electrically neutral. Among these, an amide bond, urea bond, ether bond, or ester bond is more preferably contained in the linker as a bond electrically neutral, and capable of giving hydrophilicity, from the viewpoint of inhibiting clogging due to the adsorption of platelets. From the viewpoint of the stability of the bonding, an amide bond or an ester bond is still more preferably contained, and an amide bond is particularly preferable.

The amide bond contained in the linker may be any amide bond of a primary amide, secondary amide, and tertiary amide, and a secondary amide is preferable. In addition, the position at which the amide bond contained in the linker is bound is not particularly limited. From the viewpoint of suitably inhibiting the hydrophobicity of an aromatic hydrocarbon group prone to adsorb platelets, the amide bond is preferably covalently bound to an aromatic ring of the first polymer via an alkylene group. The alkylene group is preferably methylene, ethylene, propylene, or the like, more preferably a methylene group.

In addition, to make it easier to control the bonding of the second polymer, the linker may contain an ionic functional group, preferably, for example, an amino group or a carboxylic group, more preferably an amino group from the viewpoint of blood compatibility.

To achieve both the fixation to the first polymer and the fixation to the second polymer, an electrically neutral chemical bond and an ionic functional group may be simultaneously contained as the chemical structure in the linker. In this case, it is preferable that the chemical structure contains an electrically neutral chemical bond on the first polymer side, and contains an ionic functional group on the surface side. For example, it is preferable that an amide bond is covalently bound to an aromatic ring of the first polymer via an alkylene group, and that an amino group is further bound to the terminal side.

The method of fixing the first polymer and the linker by covalent bonding is not limited, and for example, it is preferable that an aromatic ring contained in a repeating unit of the first polymer and a linker are bound to each other by carboncarbon bonding.

The carbon number of the linker is not limited, and the carbon number is preferably, for example, 2 or more and 30 or less, more preferably 4 or more and 20 or less, still more preferably 6 or more and 15 or less, to have a suitable distance between the first polymer and the second polymer, and to have the motility of the linker within a preferable range, thus making the second polymer more fixable.

A method of fixing the second polymer physically is not particularly limited. For example, in a preferable method of fixation, a copolymer containing a repeating unit having hydrophobicity is used as the second polymer, and the hydrophobic interaction of the structure is allowed to aggregate the first polymer, or the second polymer is mixed during the formation of the first polymer to be fixed by entanglement. From the viewpoint of achieving the capability of the blood component adsorbent material, the method in which the hydrophobic interaction is allowed to aggregate the first polymer is preferable.

The ratio of content of the second polymer in the surface of the blood component adsorbent material, if too small, causes no interaction between the blood component adsorbent material and blood corpuscles, thus not achieving the blood component adsorption capability, and, if too large, increases hydrophilicity too much, thus decreasing the blood component adsorption capability. Because of this, the ratio of content of the second polymer in the surface of the blood component adsorbent material is 3 mg/g or more and 30 mg/g or less, more preferably 3 mg/g or more and 20 mg/g or less.

The ratio of content of the second polymer in the blood component adsorbent material as a uniformized whole is not limited. To enhance the adsorption capability of the humoral factor of the blood component, the ratio of content of the second polymer in the blood component adsorbent material as a uniformized whole is preferably 0.3 mg/g or less.

The particle size of the blood component adsorbent material is not limited. To make blood more flowable during the adsorption of a blood component, the particle size is preferably 10 µm or more and 10 mm or less, more preferably 50 µm or more and 3 mm or less, still more preferably 100 µm or more and 1 mm or less.

The volume of pores per 1 g of the blood component adsorbent material means the volume of micropores having a diameter of 200 nm or less, the volume being determined by measuring the degree of depression of the freezing point by differential scanning calorimetry using a differential scanning calorimeter (hereinafter referred to as DSC), in which the depression is due to the capillary condensation of water in the pores.

The volume of pores per 1 g is determined as follows: a differential thermal (DSC) curve of a subject material in a hydrous state is obtained by the below-described DSC method; from the resulting DSC curve, the melting point and abundance of water are then calculated; and furthermore, the volume of pores is calculated. The material in an amount of 1 g is impregnated in 10 mL of pure water impregnated in water, and a container having the solution therein is treated in an ultrasonic cleaning device, and simultaneously degassed with an aspirator for 10 minutes. Immediately before the DSC measurement, approximately 6 mg of the resulting material in a hydrous state is taken out, and water adhering to the surface is removed to a degree to which no water droplet can be seen by excessive visual observation. Next, an aluminium-made hermetically sealed sample container is set in DSC Q100 (manufactured by TA Instruments, Inc.) that is preliminarily calibrated for temperature and calorie (the melting point, 0.0°C; the amount of heat of melting, 79.7 cal/g) with pure water and, a blank weight is measured. Subsequently, approximately 6 mg of the material is sampled, enclosed in an aluminium-made hermetically sealed sample container, and used as a measurement sample. The measurement sample is placed in DSC Q100, and the weight is measured. A value obtained by subtracting the blank weight from the weight obtained is regarded as the weight of the sample. The measurement sample is rapidly cooled to -55°C, and heated to 5°C at 0.3°C/minute. A differential scanning calorie is measured, and a DSC curve is obtained with the peak top temperature as the melting point. Then, the sample is taken out, vacuum-dried at 110°C for 2 hours, and again placed in the DSC Q100. The weight is measured, and the amount of the decrease caused between before and after the vacuum drying is regarded as the total amount of water. From the resulting DSC curve, total amount of water, and weight of the sample, the volume of pores per 1 g of the material is calculated in accordance with a method of Ishikiriyama et al. (JOURNAL OF COLLOID AND INTERFACE SCIENCE, 1995, Vol. 171, pp. 103-111).

The fact that the porous particles are composed only of the first polymer can be verified as follows: porous particles are crushed in a mortar so as be insertable into an NMR tube; a solid ¹³CNMR measurement is made; and a peak is found in an aromatic ring-derived region (δ: 110 to 170 ppm).

The fact that the second polymer is contained in the surface of the blood component adsorbent material can be verified by the fact that an absorption wavelength (3340 cm⁻¹) of the OH stretching is present in an infrared absorption spectrum of the surface of the blood component adsorbent material.

The ratio of content of the second polymer in the blood component adsorbent material as a uniformized whole can be determined as follows: the dry weight of the blood component adsorbent material is preliminarily measured; the material is then impregnated with tetrahydrofuran, hydrochloric acid, and an aqueous sodium hydroxide solution at 90°C or more overnight each; then, the material is repeatedly washed with ion-exchanged water to become neutral, and dried again; the weight of the blood component adsorbent material is measured; the weight of the blood component adsorbent material after the elution can be calculated by rounding, to one decimal place, a value obtained in accordance with the following formula 1. Ratio of content (mg/g) of second polymer in blood component adsorbent material as uniformized whole = {dry weight (1000 mg) - weight after elution (mg)} / 1 g

The ratio of content of the second polymer in the surface of the blood component adsorbent material is calculated by rounding off, to whole numbers, a value obtained in accordance with the following formula 2 on the basis of the following: the absorption intensity of a peak (2920 cm⁻¹) derived from the CH stretching on each of the infrared absorption spectrum of the blood component adsorbent material as a uniformized whole and the infrared absorption spectrum of the surface of the blood component adsorbent material; and the absorption intensity of a peak (3340 cm⁻¹) derived from the OH group on each of the infrared absorption spectrum of the blood component adsorbent material as a uniformized whole and the infrared absorption spectrum of the surface of the blood component adsorbent material. In this regard, the value of the "ratio of content of the second polymer in the blood component adsorbent material as one uniformized whole" to be used for the formula 2 was calculated using the value calculated in accordance with the formula 1, in which the value is a value not yet rounded to one decimal place. Ratio of content (mg/g) of second polymer in surface of blood component adsorbent material = ratio of content (mg/g) of second polymer in blood component adsorbent material as uniformized whole × {absorption intensity of peak derived from OH group on surface of blood component adsorbent material / absorption intensity of peak derived from CH stretching on surface of blood component adsorbent material} / {absorption intensity of peak derived from OH group in blood component adsorbent material as uniformized whole / absorption intensity of peak derived from CH stretching in blood component adsorbent material as uniformized whole}

The particle size of the blood component adsorbent material means the average values of the diameters measured as follows: 10 sample particles are taken randomly; a photograph of each sample is taken using a scanning electron microscope at a magnification of 1000 times to 3000 times; and the diameters of 10 portions per photograph (a total of 100 portions) are measured.

The "blood component adsorbent material" means a material having the capability to adsorb a blood component.

The "blood component" refers to a component constituting blood, and classified into humoral factors in blood and cells in blood. The blood component to be adsorbed by a blood component adsorbent material in the present embodiment is not particularly limited. Among the blood components, cells in blood are preferable, and it is more preferable that cells in blood and humoral factors in blood can be adsorbed simultaneously.

The "cells in blood" mean cells contained in blood, and examples thereof include: granulocytes; leucocyte components such as monocytes and lymphocytes; erythrocytes; platelets; and the like. In a case where an inflammatory disease is a subject of treatment, leucocyte components are preferable as an object to be adsorbed. Among the leucocyte components, monocytes and granulocytes are preferably removed, and activated granulocytes, activated monocytes, activated granulocyte-activated platelet complexes, or activated monocyte-activated platelet complexes are more preferable.

The "activated granulocyte" and "activated monocyte" mean a granulocyte and a monocyte that release cytokines, active oxygen, or the like by virtue of cytokines, LPS, or the like. The degree of activation can be determined by measuring the amount of activated oxygen released by activated leucocytes, or measuring the expression of a surface antigen by flow cytometry or the like.

The "activated platelet" means a platelet that releases cytokines, active oxygen, or the like owing to cytokines, LPS, or the like.

The "activated granulocyte-activated platelet complex" and "activated monocyte-activated platelet complex" mean a complex in which an activated granulocyte or activated monocyte is bound to an activated platelet, and which has phagocytosis into the self-tissue. In particular, in treatment of a patient with an inflammatory disease, it is conceivably necessary to remove an activated granulocyte-activated platelet complex that is considered to be directly involved in the pathology.

The granulocytes are further classified into neutrophils, basophils, and eosinophils, and it is preferable that, without selectivity thereamong, components derived from monocytes and granulocytes are removed all together.

The "humoral factor in blood" refers to an organic substance dissolved in blood. Specific examples include: proteins such as urea, β2-microglobulin, cytokine, IgE, and IgG; and polysaccharides such as lipopolysaccharide (LPS). Among these, proteins such as urea and cytokine and polysaccharides such as LPS are preferable as an object to be adsorbed, and furthermore, in a case where an inflammatory disease is a subject of treatment, cytokines are more preferable as an object to be adsorbed.

A "cytokine" means a group of proteins that are produced from various cells, for example, immunocompetent cells, by a stimulus such as infection or injury, are released out of cells, and act. Examples thereof include interferon α, interferon β, interferon γ, interleukin 1 to interleukin 15, tumor necrosis factor-α, tumor necrosis factor-β, high-mobility group box-1, erythropoietin, and monocyte chemotactic factor, and particularly in the treatment of an inflammatory disease, interleukin 8 (IL-8) is preferable as an object to be adsorbed.

The "inflammatory disease" refers to the whole of diseases that initiate an inflammatory reaction in the body. Examples thereof include systemic lupus erythematosus, malignant rheumatoid arthritis, multiple sclerosis, ulcerative colitis, Crohn's disease, drug-induced hepatitis, alcoholic hepatitis, hepatitis A, hepatitis B, hepatitis C, hepatitis D, hepatitis E, sepsis (for example, gram-negative bacteria-derived sepsis, gram-positive bacteria-derived sepsis, culture-negative sepsis, and fungal sepsis), influenza, acute respiratory distress syndrome (ARDS also referred to as acute respiratory distress syndrome or acute respiratory distress syndrome), acute lung injury (ALI), pancreatititis, idiopathic pulmonary fibrosis (IPF), inflammatory enteritis (for example, ulcerative colitis and Crohn's disease), blood preparation transfusion, organ transplantation, reperfusion damage after organ transplantation, cholecystitis, cholangitis, newborn blood group incompatibility, and the like.

Among the inflammatory diseases, drug-induced hepatitis, alcoholic hepatitis, hepatitis A, hepatitis B, hepatitis C, hepatitis D or hepatitis E, sepsis (for example, gram-negative bacteria-derived sepsis, gram-positive bacteria-derived sepsis, culture-negative sepsis, and fungal sepsis), influenza, acute respiratory distress syndrome, acute lung injury, pancreatititis, and idiopathic pulmonary fibrosis are preferable as an object to be treated, because such a disease causes a causative agent to be released in blood, and can be expected to receive particularly an effect of treatment by blood purification. Preferable examples of uses for a column for adsorption in the present embodiment include therapeutic uses for the above-described inflammatory diseases. Among these, therapeutic uses for sepsis (for example, gram-negative bacteria-derived sepsis, gram-positive bacteria-derived sepsis, culture-negative sepsis, and fungal sepsis), influenza, acute respiratory distress syndrome, acute lung injury, and idiopathic pulmonary fibrosis are more preferable, because these diseases are difficult to treat with a drug alone, and are considered to be diseases in which both a cytokine and an activated leucocyte-activated platelet are involved.

"Adsorption" means a state in which a specific substance adheres to a material, and is not detached easily. The principle of adsorption is not particularly limited. Adsorption means, for example, a state of adhesion due to a van der Waals force, for example, ionic interaction such as electrostatic interaction, hydrophobic interaction, or hydrogen bonding, or a state of biological adhesion such as the adhesion of a cell or the phagocytosis of a leucocyte. A blood component adsorbent material according to the present embodiment is preferably able to perform adsorption by a van der Waals force.

A blood component adsorbing material according to the present embodiment can be produced, for example, by the below-described method, but is not limited to this method.

The production of porous particles composed only of the first polymer can be achieved by adding a monomer having an aromatic hydrocarbon group, a cross-linking agent, a dispersant, and an initiator in suspension polymerization. Examples of the monomer include styrene, ethylvinylbenzene, or divinylbenzene, which is a vinyl monomer having an aromatic ring, and it is preferable to contain at least divinylbenzene. Examples of the cross-linking agent include divinylbenzene, examples of the dispersant include polyvinyl alcohol, and examples of the initiator include benzoyl peroxide.

As the vinyl monomer, a commercially available vinyl monomer can be used directly. A commercially available divinylbenzene monomer is commonly a mixture containing ethylvinylbenzene, and the first polymer obtained by polymerization of divinylbenzene is poly(ethylvinylbenzene/divinylbenzene) that is a copolymer.

The particle size of the porous particle can be made smaller by increasing the concentration of the dispersant.

The pore volume of porous particles can be increased by decreasing the monomer concentration, increasing the initiator concentration, or decreasing the cross-linking agent concentration (for example, the divinylbenzene concentration).

A method of fixing the second polymer to porous particles can be performed, for example, by the following: a method in which the second polymer is dissolved in an organic solvent, and porous particles are added in or coated with the solution; or a method in which an amine compound is fixed to the surfaces of porous particles, and allowed to react as a linker with the terminal structure of the second polymer.

In a case where the second polymer is dissolved in an organic solvent, and where porous particles are added in or coated with the solution, a preferable organic solvent is *N,N*-dimethyl formamide, diethyl ether, dioxane, tetrahydrofuran, dimethyl sulfoxide, toluene, xylene, hexane, or ethyl acetate; and *N,N*-dimethyl formamide, tetrahydrofuran, dimethyl sulfoxide, toluene, or ethyl acetate is particularly preferable.

The concentration of the second polymer in the organic solvent is preferably 1 to 10%, more preferably 5%.

The second polymer to be used can be a commercially available product.

In a case where an amine compound is fixed to the surfaces of porous particles, and allowed to react as a linker with the terminal structure of the second polymer, for example, a hydroxy group, carboxylic acid, epoxy group, urea group, anhydrous carboxylic group, or the like as the terminal structure of the second polymer is fixed, and allowed to condense with an amine or carboxylic acid of the amine compound, so that the fixation can be achieved.

The amine compound can be fixed to the porous particles by further fixing a functional group as a linker to the aromatic ring contained in the first polymer. The functional group as a linker is not limited, and is, for example, an alkyl halide group, carboxylic group, epoxy group, or the like.

The amine compound to be fixed is preferably, for example, ethylenediamine, diethylenetriamine, triethylenetetramine, polyethyleneimine, glycine, or the like, and is preferably ethylenediamine or glycine. As these, commercially available products can be used.

The reaction solvent is preferably, for example, *N,N*-dimethyl formamide, diethyl ether, dioxane, tetrahydrofuran, or dimethyl sulfoxide, more preferably dimethyl sulfoxide.

The base is, for example, an organic base such as triethylamine or 1,4-diazabicyclo[2.2.2]octane, or an inorganic base such as sodium hydroxide, preferably an organic base such as triethyl amine.

The concentration of the amine compound in a reaction liquid is preferably 50 mM to 1000 mM, more preferably 50 mM to 100 mM.

The fixation of an alkyl halide group can be produced, for example, by incorporating a hydroxyalkyl halide compound into porous particles in the presence of a catalyst.

The hydroxyalkyl halide compound is preferably, for example, N-hydroxymethyl-2-chloroacetamide, or the like, and as the compound, a commercially available product can be used.

The reaction solvent is, for example, nitrobenzene, nitropropane, chlorobenzene, toluene, or xylene, preferably nitrobenzene or nitropropane.

The catalyst is, for example, a Lewis acid such as sulfuric acid, hydrochloric acid, nitric acid, aluminium halide (III) (for example, aluminium chloride (III)), or iron halide (III) (for example, iron chloride (III)), preferably sulfuric acid.

The concentration of the catalyst in the reaction liquid is preferably 5 wt% to 80 wt%, more preferably 30 wt% to 70 wt%.

The reaction temperature is preferably 0°C to 90°C, more preferably 5°C to 40°C.

The reaction time is preferably 1 minute to 120 hours, more preferably 5 minutes to 24 hours.

For coating with the second polymer, the amount of fixation to the second polymer in the surface of the blood component adsorbent material and the amount of fixation to the second polymer in the blood component adsorbent material as a uniformized whole can be controlled to be increased by increasing the concentration of the second polymer in the organic solvent, increasing the coating temperature, or decreasing the molecular weight of the second polymer.

In a case where the second polymer is fixed by a chemical reaction, the amount of fixation to the second polymer in the surface of the blood component adsorbent material and the amount of fixation to the second polymer in the blood component adsorbent material as a uniformized whole can be controlled by increasing the concentration of the second polymer during the fixation, increasing the temperature, or allowing the second polymer to react for a long time.

The above-described blood component adsorbent material is preferably used as a material for packing a container that forms the external shape of an adsorption column.

The container that forms the external shape of an adsorption column can be in the form of a container that can be packed with a blood component adsorbent material, and has an inlet and an outlet for blood. The container is, for example, a cylindrical container or a prismatic container such as a triangle-pole-like, quadrangular-prism-like, hexagonal-prism-like, or octagonal-prism-like container.

Furthermore, the adsorption column including the above-described blood component adsorbent material can be suitably used for treatment of a bacteria-derived infection and for extracorporeal circulation for use in the treatment of a bacteria-derived infection. For use in the treatment of a bacteria-derived infection, an extracorporeal circulation method is preferable, in which an adsorption column including the blood component adsorbent material and a patient are connected with a blood circuit, and the bodily fluid taken out of the patient is passed through the adsorption column, and returned to the patient. The time for treating the bodily fluid or the like is preferably continuous, more preferably 4 hours or more, still more preferably 24 hours or more, from the viewpoint of inhibiting further induction of inflammation due to the blood component.

The adsorption column including the adsorbent material may be used in combination with another bodily fluid treating method or medical device. Examples of the another bodily fluid treating method and medical device include plasma exchange, peritoneal dialysis, plasma separator, hemofilter, artificial hearts and lungs, or ECMO.

Examples of a method of evaluating the adsorption capability of the blood component adsorbent material include a method of evaluating the ratio of adsorption of leucocytes. Examples of a method of calculating the ratio of adsorption of leucocytes include a method in which a container having an inlet and an outlet is packed with the blood component adsorbent material, a liquid containing leucocytes is passed through the container, and the ratio of adsorption of leucocyte is calculated from a change in the concentration between the inlet and the outlet. The leucocyte concentration can be measured using a commercially available multi-channel blood corpuscle analyzer, and can be measured specifically by the below-described method.

A column is produced, which is a cylindrical column (1 cm in inner diameter × 5.14 cm in height) having an inlet and an outlet for blood in the upper and lower portions thereof, and packed with 3.1 mL of the blood component adsorbent material. Through this column, human blood having a temperature kept at 37°C (outside temperature) is passed through at a flow rate of 1.9 mL/min for 5 minutes. After the 5 minutes, the blood at the inlet of the column and the blood components at the outlet of the column are analyzed, and the ratio of adsorption of leucocytes by the blood component adsorbent material is calculated. In this regard, the number of blood components of each kind is measured using a multi-channel automatic blood corpuscle analyzer XT-1800i (manufactured by Sysmex Corporation). The ratio of adsorption of leucocytes can be calculated in accordance with the following formula 3. Ratio (%) of adsorption of leucocytes by blood component adsorbent material = {(total number of granulocytes and monocytes in blood at inlet of column) - (total number of granulocytes and monocytes in blood at outlet of column)} / (total number of granulocytes and monocytes in blood at inlet of column) × 100

A leucocyte is a cell, and, from the viewpoint of involving unevenness of measurement in the ratio of adsorption, having the ratio of adsorption at 20% or more makes it possible to determine that the removal has been achieved significantly.

For the above-described reason, the ratio of adsorption of 20% or more makes it possible to consider that the leucocyte adsorption capability is achieved sufficiently, and a ratio of adsorption of 50% or more is still more preferable.

In addition, a blood component adsorbent material according to the present invention adsorbs leucocytes by a van der Waals force, and thus, a difference in the ratio of removal derived from the leucocyte components is considered to be small. Accordingly, having the ratio of adsorption of leucocytes at 20% or more makes it possible to consider that 20% or more of each of the individual leucocyte components, namely monocytes, neutrophils, and granulocytes, has been removed, and that the removal has been achieved significantly, even taking the unevenness of measurement into account.

Among the adsorption capabilities of the blood component adsorbent material, a method of evaluating the ratio of adsorption of each of a monocyte, neutrophil, basophil, and eosinophil can be performed by the below-described procedures 1) to 4).
1) A column is produced, which is a cylindrical column (1 cm in inner diameter × 5.14 cm in height) having an inlet and an outlet for blood in the upper and lower portions thereof, and packed with 3.1 mL of a blood component adsorbent material 4.
2) Through this column, human blood having a temperature kept at 37°C (outside temperature) is passed through at a flow rate of 1.9 mL/min for 5 minutes.
3) After the 5 minutes, the blood at the inlet of the column and the blood at the outlet of the column are sampled, and the value of each of the number of monocytes, the number of neutrophils, the number of basophils, and the number of eosinophils is obtained using a multi-channel automatic blood corpuscle analyzer XT-1800i (manufactured by Sysmex Corporation).
4) Each of the values obtained in accordance with the following formulae 4 to 7 is rounded off to whole numbers, and the ratio of removal of monocytes, the ratio of removal of neutrophils, the ratio of removal of basophils, and the ratio of removal of eosinophils by the blood component adsorbent material are calculated. Ratio (%) of adsorption of monocytes by blood component adsorbent material = {(number of monocytes in blood at inlet of column) - (number of monocytes in blood at outlet of column)} / (number of monocytes at inlet of column) × 100 Ratio (%) of adsorption of neutrophils by blood component adsorbent material = {(number of neutrophils in blood at inlet of column) - (number of neutrophils in blood at outlet of column)} / (number of neutrophils at inlet of column) × 100 Ratio (%) of adsorption of basophils by blood component adsorbent material = {(number of basophils in blood at inlet of column) - (number of basophils at outlet of column)} / (number of basophils in blood at inlet of column) × 100 Ratio (%) of adsorption of eosinophils by blood component adsorbent material = {(number of eosinophils in blood at inlet of column) - (number of eosinophils at outlet of column)} / (number of eosinophils in blood at inlet of column) × 100

Another example is a method in which the blood component adsorbent material is impregnated with fetal bovine serum (hereinafter referred to as FBS) in which cytokines are dissolved, the amount of a decrease in the concentration of cytokines in FBS after the impregnation is evaluated, and the ratio of adsorption of cytokines is calculated. A cytokine is a substance that is preferably removed from blood to improve the pathology of an inflammatory disease, and thus, it can be considered that the larger the amount of decrease in the concentration caused by the impregnation, the higher the blood component adsorption capability. Examples of a cytokine include interleukin 1β, interleukin 6, interleukin 8, high-mobility group protein-1, tumor necrosis factor-β, and the like. Interleukin 6 and interleukin 8 are more preferable from the viewpoint of being a typical biomarker in the therapeutic practice of an inflammatory disease.

The adsorption of cytokines into the blood component adsorbent material is considered to be an equilibrium reaction derived from an intermolecular force such as a van der Waals force, and thus does not depend on the concentration of cytokines. It is considered that performing an adsorption treatment for approximately 4 hours achieves the adsorption equilibrium.

For the above-described reason, all of the ratios of adsorption of cytokines are preferably 100% in 4 hours, and because of the time dependence, having the ratio at 50% or more in 2 hours makes it possible to consider that the adsorption capability has been achieved sufficiently.

A decrease caused by sterilization in the capability of the blood component adsorbent material can be evaluated by the following procedures 1) to 5).
1) The ratio of adsorption of leucocytes by the blood component adsorbent material is measured preliminarily.
2) A blood component adsorbent material in an amount of 5 mL and distilled water in an amount of 25 mL are placed in a 50 mL centrifuge tube, and irradiated with a gamma beam at an absorbed dose of 40 kGy to obtain a blood component adsorbent material after sterilization.
3) A column packed with the resulting blood component adsorbent material after sterilization and a column packed with the blood component adsorbent material before sterilization are produced.
4) Through each column, human blood kept at 37°C (outside temperature) is passed through at a flow rate of 1.9 mL/min for 5 minutes.
5) After the 5 minutes, the blood at the inlet of the column and the blood at the outlet of the column are sampled, the ratio of adsorption of leucocytes is measured using a multi-channel automatic blood corpuscle analyzer XT-1800i (manufactured by Sysmex Corporation), a value obtained in accordance with the following formula 8 is rounded off to whole numbers, and the ratio of decrease in the capability of the blood component adsorbent material between before and after sterilization is calculated. Ratio of decrease in capability of blood component adsorbent material between before and after sterilization (%) = 100 × {(ratio of adsorption of leucocytes by blood component adsorbent material) - (ratio of adsorption of leucocytes by blood component adsorbent material after sterilization)} / (ratio of adsorption of leucocytes by blood component adsorbent material)

A preferable range of the ratio of decrease in the capability of the blood component adsorbent material between before and after sterilization is not limited, and the ratio of decrease in the capability is preferably 50% less, because this ratio can decrease the elution of a degradation product from the blood component adsorbent material.

The ratio of adsorption of platelets by the blood component adsorbent material can be evaluated by the following procedures 1) to 4).
1) A column is produced, which is a cylindrical column (1 cm in inner diameter × 5.14 cm in height) having an inlet and an outlet for blood in the upper and lower portions thereof, and packed with 3.1 mL of a blood component adsorbent material.
2) To human blood kept at 37°C (outside temperature), nafamostat mesilate as an anticoagulant agent is added so as to be at 200 µg/mL, and the resulting mixture is promptly passed through the column at a flow rate of 0.7 mL/min for 5 minutes.
3) After the 5 minutes, the blood at the inlet of the column and the blood at the outlet of the column are sampled, and the number of platelets is obtained using a multi-channel automatic blood corpuscle analyzer XT-1800i (manufactured by Sysmex Corporation).
4) A value obtained in accordance with the following formula 9 is rounded off to whole numbers to calculate the ratio of adsorption of platelets by the blood component adsorbent material. Ratio (%) of adsorption in platelets of blood component adsorbent material = 100 × {(number of platelets in blood at inlet of column) - (number of platelets in blood at outlet of column)} / (number of platelets in blood at inlet of column)

A preferable range of the ratio of adsorption of platelets is not limited, and the ratio of adsorption of platelets is preferably 50% or less, more preferably 20% or less, to prevent the generation of aggregates in blood circulation, and decrease the possibility of clogging.

### Examples

Below, a blood component adsorbent material according to the present invention will be described specifically with reference to Examples, and the present invention is not limited to these Examples.

Here, wt% means weight% in Examples. M represents mol/L, and mM represents mmol/L. Unless otherwise specified, the weight of each of a blood component adsorbent material, porous particle, first polymer, and second polymer represents a dry weight. An absorbance was measured at room temperature, using an ultraviolet and visible spectrophotometer (UV-1280, manufactured by Shimadzu Corporation). Before the measure of absorbance, a blank measurement was made preliminarily, and the peak of the background was subtracted. Unless otherwise specified, an infrared absorption spectrum was measured by the following technique.

On a Nicolet iS5 FT-IR (manufactured by Thermo Scientific; with iD5 diamond ATR accessories; the detector, DTGS KBr; the beam splitter, KBr), the Advanced ATR mode is selected, and the parameters (the number of scans, 16; the data interval, 0.241 cm⁻¹; without automated atmospheric correction) are set. After the setting, a background measurement was made. When the background measurement ended, the blood component adsorbent material preliminarily dried with a warm air drier at 60°C for 4 hours was spread over a prism, and pressed against the prism until the pressure device was locked. A measurement was started within 20 minutes after the background measurement. In this regard, the range of measurement was set at a wavenumber of 4500 cm⁻¹ to 500 cm⁻¹.

In Examples, the ratio of saponification of polyvinyl alcohol has the same meaning as the ratio of abundance of hydroxy groups in the repeating units, and the remaining repeating units not saponified are polyvinyl acetate. For example, in polyvinyl alcohol having a ratio of saponification of 10%, 90 mol% of the repeats is polyvinyl acetate, and 10 mol% is polyvinyl alcohol.

### (Setting of Reactor)

To a 500 mL three-neck flask, a stirrer, Dimroth condenser, thermocouple, and eggbeater were attached to form a reactor, and the temperature was controlled with a mantle heater with the temperature measured with a thermocouple.

### (Production of Blood Component Adsorbent Material 1)

### Produce of porous particles:

An aqueous PVA solution in an amount of 115 mL having 0.68 g of polyvinyl alcohol (hereinafter referred to as PVA) dissolved therein, 0.71 g of monosodium phosphate (hereinafter referred to as MSP), 2.36 g of disodium phosphate (hereinafter referred to as DSP), 0.01 g of trisodium phosphate (hereinafter referred to as TSP), and 115 mL of an aqueous phosphoric acid solution having 0.01 g of sodium nitrite dissolved therein were each produced. The whole amount of the aqueous PVA solution was added to the reactor, and heated to 70°C, and then, the whole amount of the aqueous phosphoric acid solution was added to form an aqueous phase. Furthermore, 106 g of divinylbenzene (hereinafter referred to as DVB), 116 g of cyclohexanol, 11 g of polypropylene glycol (hereinafter referred to as PPG), and 1.1 g of benzoyl peroxide (hereinafter referred to as BPO) were mixed to form an organic phase. The organic phase was poured into the aqueous phase in the reactor, the stirrer was started up to cause stirring, and the dispersion of the droplets was verified. The time when the temperature reached 80°C was regarded as the start of reaction, and the reaction was continued for 16 hours.

Only the solvent was decanted from the reactor, and water in the same amount as the amount of the solvent removed was added. Then, the solution was stirred for 30 minutes, and the solvent was decanted. The operation from the addition of water was repeated 5 times in total to wash the product. Next, the solvent was decanted, the same amount of methanol was added, the resulting mixture was stirred for 10 minutes, and the solvent was decanted. The operation from the addition of methanol was repeated 3 times in total. Subsequently, the product was treated in acetone in a soxhlet extraction device overnight to extract an oligomer, which was then vacuum-dried for 8 hours, impregnated with isopropyl alcohol, and then added to purified water. Finally, the resulting product was passed through a sieve to have a uniform particle size, and dried using a warm air drier at 100°C. The porous particles obtained were used as a blood component adsorbent material 1 having poly(ethylbenzene/divinylbenzene) as the first polymer and containing no linker.

The fact that the blood component adsorbent material 1 contained the first polymer was verified by the fact that a disubstituted aromatic compound-derived peak (800 cm⁻¹) of C-H out-of-plane bending vibration was present in a measurement of an infrared absorption spectrum of the surface of the blood component adsorbent material 1.

### (Production of Blood Component Adsorbent Material 2)

*N*-methylol-α-chloroacetamide (hereinafter referred to as NMCA) in an amount of 2.4 g was added to a solution mixture of 31 g of nitrobenzene and 31 g of concentrated sulfuric acid. Until NMCA was dissolved, the resulting mixture was stirred at 10°C to obtain an NMCA solution. Next, 2.0 g of nitrobenzene, 2.0 g of concentrated sulfuric acid, and 0.2 g of paraformaldehyde (hereinafter referred to as PFA) were added, and until PFA was dissolved, the resulting mixture was stirred at 20°C to obtain a PFA solution. The PFA solution in an amount of 4.2 g was cooled to 5°C, and mixed with the NMCA solution. The resulting mixture was stirred for 5 minutes, and 1 g of the blood component adsorbent material 1 was added to and impregnated with the mixture for 2 hours. The blood component adsorbent material 1 after the impregnation was immersed in 200 mL of nitrobenzene at 0°C to stop the reaction, and then, the nitrobenzene adhering to the porous particles was washed with methanol to obtain chlorinated particles.

To a solution mixture of 0.2 g of triethyl amine and 51 g of dimethyl sulfoxide (hereinafter referred to as DMSO), ethylenediamine (hereinafter referred to as EDA) was added and dissolved so as to be at a concentration of 0.8 M. Chlorinated particles were added to and impregnated with the resulting solution at 40°C for 3 hours. The particles were collected on a glass filter by filtration, and washed with 500 mL of DMSO. The particles were further washed with 60 mL of distilled water, and additionally washed with 3 L each of distilled water and physiological saline to obtain EDA-bound particles.

With 0.5 g of the EDA-bound particles obtained, 10 mg of a 4-(4,6-dimethoxy-1,3,5-triazine-2-yl)-4-methylmorpholinium=chloride n-hydrate, 10 mg of PVA (the ratio of saponification, 10%) for fixation, and 10 mL of water were mixed, and the resulting mixture was allowed to react for 24 hours. The porous particles obtained were taken out onto filter paper, and during suction filtration, 200 mL of methanol was poured onto the particles to be washed. Only the porous particles were peeled off from the filter paper using a spatula, and dried with a warm air drier at 50°C for 24 hours. The product taken out of the warm air drier was obtained as a blood component adsorbent material 2 having a structure formed as follows: an amide bond was added to the aromatic ring via a methylene group as a linker; an amino group was further added to the surface side; and an amide bond was further added to the surface side via an ethylene group.

The fact that the blood component adsorbent material 2 contained the second polymer was verified by the fact that a hydroxy group-derived peak (3340 cm⁻¹) was present in a measurement of an infrared absorption spectrum of the surface of the blood component adsorbent material 2.

### (Production of Blood Component Adsorbent Material 3)

A blood component adsorbent material 3 was obtained by performing the production in accordance with all the same procedures as for the blood component adsorbent material 2 except that the ratio of saponification of the PVA (the ratio of saponification, 10%; the degree of polymerization, 500) for fixation was changed from 10% to 35%.

### (Production of Blood Component Adsorbent Material 4)

A blood component adsorbent material 4 was obtained by performing the production in accordance with all the same procedures as for the blood component adsorbent material 2 except that the ratio of saponification of the PVA (the ratio of saponification, 10%; the degree of polymerization, 500) for fixation was changed from 10% to 95%.

### (Production of Blood Component Adsorbent Material 5)

A blood component adsorbent material 5 was obtained by performing the production in accordance with all the same procedures as for the blood component 4 except that the added amount of the PVA for fixation was changed from 10 mg to 3 mg.

### (Production of Blood Component Adsorbent Material 6)

A blood component adsorbent material 6 was obtained by performing the production in accordance with all the same procedures as for the blood component 4 except that the added amount of the PVA for fixation was changed from 10 mg to 20 mg.

### (Production of Blood Component Adsorbent Material 7)

A blood component adsorbent material 7 was obtained by performing the production in accordance with all the same procedures as for the blood component 4 except that the added amount of the PVA for fixation was changed from 10 mg to 25 mg.

### (Production of Blood Component Adsorbent Material 8)

A blood component adsorbent material 5 was obtained by performing the production in accordance with all the same procedures as for the blood component 4 except that the added amount of the PVA for fixation was changed from 10 mg to 5 mg.

### (Production of Blood Component Adsorbent Material 9)

Porous particles in an amount of 1 g obtained by the same method as the blood component adsorbent material 1 and 1 g of poly(ethylene-vinyl alcohol) (the ratio of content of the polyethylene repeating unit, 40%; the degree of polymerization, 250) were added in 20 mL of toluene, and the resulting mixture was stirred for 2 hours. The porous particles obtained were taken out onto filter paper, and during suction filtration, 200 mL of methanol was poured onto the particles, which were thus washed. Only the porous particles were peeled off from the filter paper using a spatula, and dried with a warm air drier at 50°C for 24 hours. A blood component adsorbent material 9 was obtained, which contained no linker, and had poly(ethylene-vinyl alcohol) (the ratio of content of the polyethylene repeating unit, 40%) fixed therein by physical fixation.

### (Production of Blood Component Adsorbent Material 10)

An aqueous PVA solution in an amount of 115 mL having 0.6 g of PVA dissolved therein, 0.71 g of MSP, 2.4 g of DSP, 0.01 g of TSP, and 115 mL of an aqueous phosphoric acid solution having 0.01 g of sodium nitrite dissolved therein were each produced. The whole amount of the aqueous PVA solution was added to the reactor, and heated to 70°C, and then, the whole amount of the aqueous phosphoric acid solution was added to form an aqueous phase. Furthermore, 106 g of DVB, 116 g of cyclohexanol, 6.0 g of PPG, 1.1 g of BPO, and 3.0 g of PVA (the ratio of saponification, 95%; the degree of polymerization, 500) were mixed to obtain an organic phase. The organic phase was poured into the aqueous phase in the reactor, the stirrer was started up to cause stirring, and the dispersion of the droplets was verified. The time when the temperature reached 80°C was regarded as the start of reaction, and the reaction was continued for 16 hours.

Only the solvent was decanted from the reactor, and water in the same amount as the amount of the solvent removed was added. Then, the solution was stirred for 30 minutes, and the solvent was decanted. The operation from the addition of water was repeated 5 times in total to wash the product. Next, the solvent was decanted, the same amount of methanol was added, the resulting mixture was stirred for 10 minutes, and the solvent was decanted. The operation from the addition of methanol was repeated 3 times in total. Subsequently, the product was treated in acetone in a soxhlet extraction device overnight to extract an oligomer, which was then vacuum-dried for 8 hours to obtain a blood component adsorbent material 10 which contained no linker, and had PVA fixed therein by physical fixation.

### (Production of Blood Component Adsorbent Material 11)

Chlorinated particles were produced by the same procedures as for the blood component adsorbent material 2. Furthermore, to a solution mixture of 0.2 g of triethyl amine and 51 g of DMSO, glycine (hereinafter referred to as GL) was added and dissolved so as to be at a concentration of 0.8 M. Chlorinated particles were added to and impregnated with the resulting mixture at 40°C for 3 hours. The particles were collected on a glass filter by filtration, and washed with 500 mL of DMSO. The particles washed with DMSO were further washed with 60 mL of distilled water, and washed with 3 L each of distilled water and physiological saline to obtain GL-bound particles.

The resulting GL-bound particles in an amount of 0.5 g, 10 mg of 1-ethyl-3-(3-dimethylaminopropyl)carbodiimide hydrochloride, 10 mg of poly(2-hydroxyethyl methacrylate) (hereinafter referred to as PHEMA; the degree of polymerization, 200), and 10 mL of water were mixed, and the resulting mixture was allowed to react for 24 hours. The porous particles obtained were taken out onto filter paper, and during suction filtration, 200 mL of methanol was poured onto the particles to be washed. From the filter paper, only the porous particles were collected using a spatula, and dried using a warm air drier at 50°C for 24 hours to obtain a blood component adsorbent material 11 having PHEMA fixed therein, and having a structure formed as follows: an amide bond was added to the aromatic ring via a methylene group as a linker; an amino group was further added to the surface side; and an ester group was further added to the surface side via an ethylene group.

### (Production of Blood Component Adsorbent Material 12)

GL-bound particles were produced by the same procedures as for the blood component adsorbent material 11. Furthermore, the GL-bound particles in an amount of 0.5 g, 10 mg of 1-ethyl-3-(3-dimethylaminopropyl)carbodiimide hydrochloride, 10 mg of dextran sulfate (the degree of polymerization, 30), and 10 mL of water were mixed, and the resulting mixture was allowed to react for 24 hours. The porous particles obtained were taken out onto filter paper, and during suction filtration, 200 mL of methanol was poured onto the particles to be washed. Only the porous particles were peeled off from the filter paper using a spatula, and dried with a warm air drier at 50°C for 24 hours. The porous particles were taken out of the warm air drier to obtain a blood component adsorbent material 12 which had dextran sulfate fixed therein, and had a structure formed as follows: an amide bond was added to the aromatic ring via a methylene group as a linker; an amino group was further added to the surface side; and an ester group was further added to the surface side via an ethylene group.

### (Production of Blood Component Adsorbent Material 13)

A blood component adsorbent material 13 having PVA fixed therein was obtained by performing the production in accordance with the same procedures as for the blood component adsorbent material 4 except that, in the production of porous particles, 120 g of DVB as an organic phase, 80 g of toluene, 90 g of isooctane, and 1.1 g of BPO were mixed and adjusted.

### (Production of Blood Component Adsorbent Material 14)

A blood component adsorbent material 14 having PVA fixed therein was obtained by performing the production in accordance with the same procedures as for the blood component adsorbent material 4 except that, in the production of porous particles, 130 g of DVB as an organic phase, 116 g of cyclohexanol, 11 g of PPG), and 1.3 g of benzoyl peroxide (BPO) were mixed and adjusted.

### (Production of Blood Component Adsorbent Material 15)

A blood component adsorbent material 15 having PVA fixed therein was obtained by performing the production in accordance with the same procedures as for the blood component adsorbent material 4 except that, in the production of porous particles, 83 g of DVB as an organic phase, 116 g of cyclohexanol, 38 g of PPG, and 0.8 g of BPO were mixed and adjusted.

### (Production of Blood Component Adsorbent Material 16)

A blood component adsorbent material 16 was obtained by performing the production in accordance with all the same procedures as for the blood component adsorbent material 2 except that the PVA (the ratio of saponification, 10%) for fixation was changed to the PVA (the ratio of saponification, 100%; the degree of polymerization, 10000) for fixation, and that the solvent to which EDA-bound particles were added was changed from water to isopropanol.

### (Production of Blood Component Adsorbent Material 17)

A blood component adsorbent material 17 was obtained by performing the production in accordance with the same procedures as for the blood component adsorbent material 11 except that poly(2-hydroxyethyl methacrylate) was changed to poly(4-hydroxybutyl acrylate) (the degree of polymerization, 80).

### (Production of Blood Component Adsorbent Material 18)

A blood component adsorbent material 18 was obtained by performing the production in accordance with the same procedures as for the blood component adsorbent material 12 except that dextran sulfate was changed to lactose.

### (Production of Blood Component Adsorbent Material 19)

A blood component adsorbent material 19 was obtained by performing the production in accordance with the same procedures as for the blood component adsorbent material 12 except that dextran sulfate was changed to dextran (the degree of polymerization, 250).

### (Production of Blood Component Adsorbent Material 20)

### Production of polystyrene porous particles:

Polystyrene porous particles in which the first polymer was poly(styrene/ethylvinylbenzene/divinylbenzene) were obtained by performing the production in accordance with the same procedures as for the blood component adsorbent material 1 except that 106 g of DVB to be added was changed to a mixture of 105 g of styrene and 1 g of DVB, that the added amount of PPG was changed from 11 g to 14 g, and that the added amount of BPO was changed from 1.1 g to 0.7 g.

A blood component adsorbent material 20 was obtained by performing the production in accordance with the same procedures as for the blood component adsorbent material 4 except that the porous particles to be used were changed to polystyrene porous particles.

### (Production of Blood Component Adsorbent Material 21)

### Production of hydroxy group-containing porous particles:

Epoxy group-containing porous particles were obtained by performing the production in accordance with the same procedures as for the blood component adsorbent material 1 except that 106 g of DVB to be added was changed to a mixture of 90 g of DVB and 16 g of glycidyl ether containing no aromatic hydrocarbon group. The epoxy group-containing porous particles in an amount of 1 g were added to 50 mL of a 6 N aqueous sodium hydroxide solution, heated at 60°C for 24 hours to open the ring of the epoxy group, and converted to porous particles containing a diol group having two hydroxy groups. Only the particles were taken out of the solution, and repeatedly washed with water until the wash was no longer colored by phenolphthalein. The diol group-containing porous particles which contained neither the linker after washing nor the second polymer, in which the first polymer was poly(ethylvinylbenzene/divinylbenzene/glycidyl ether ring-opened product), and in which a repeating unit containing no aromatic hydrocarbon group was present in the first polymer were obtained as a blood component adsorbent material 21.

### (Production of Blood Component Adsorbent Material 22)

### Production of chloromethyl group-containing porous particles:

Chloromethyl group-containing porous particles in which the first polymer was poly(ethylvinylbenzene/divinylbenzene/chloromethylstyrene) were obtained by performing the production in accordance with the same procedures as for the blood component adsorbent material 1 except that 106 g of DVB to be added was changed to a mixture of 36 g of chloromethylstyrene and 70 g of DBV.

A blood component adsorbent material 22, in which an amino group was added via a methylene group as a linker, and in which an ester group was further added to the surface side via a methylene group, was obtained by performing the production in accordance with the same procedures as for the blood component adsorbent material 11 except that the chlorinated particles were changed to chloromethylated particles, and that poly(2-hydroxyethyl methacrylate) was changed to polyvinyl alcohol (the ratio of saponification, 95%; the degree of polymerization, 500).

### Measurement of pore volume of blood component adsorbent material 1:

The blood component adsorbent material 1 in an amount of 1 g was impregnated with 10 mL of pure water, and a container having the solution therein was treated in an ultrasonic cleaning device, and simultaneously degassed with an aspirator for 10 minutes. Immediately before the DSC measurement, the blood component adsorbent material 1 obtained in a hydrous state was taken out, and water adhering to the surface was removed to a degree to which no water droplet could be seen by visual observation. Next, an aluminium-made hermetically sealed sample container was set in DSC Q100 (manufactured by TA Instruments, Inc.) that was preliminarily calibrated for temperature and calorie (the melting point, 0.0°C; the amount of heat of melting, 79.7 cal/g) with pure water and, a blank weight was measured. Subsequently, approximately 6 mg of the blood component adsorbent material 1 was sampled, enclosed in an aluminium-made hermetically sealed sample container, and used as a measurement sample. The measurement sample was placed in DSC Q100, and the weight is measured. A value obtained by subtracting the blank weight from the weight obtained was regarded as the weight of the sample. Subsequently, the measurement sample was rapidly cooled to -55°C, and heated to 5°C at 0.3°C/minute. A differential scanning calorie was measured, and a DSC curve was obtained. Then, the sample was taken out, vacuum-dried at 110°C for 2 hours, and again placed in the DSC Q100. The weight was measured, and the amount of the decrease caused between before and after the vacuum drying was regarded as the total amount of water. From the resulting DSC curve, total amount of water, and weight sample, the volume of pores per 1 g of the blood component adsorbent material 1 was calculated in accordance with a method of Ishikiriyama et al. (JOURNAL OF COLLOID AND INTERFACE SCIENCE, 1995, Vol. 171, pp. 103-111). The results are tabulated in Table 1.

### (Measurement of pore volume of each of blood component adsorbent materials 2 to 15)

The pore volume per 1 g of each of the blood component adsorbent materials 2 to 15 was measured in the same manner as the pore volume per 1 g of the blood component adsorbent material 1 was measured. The results are tabulated in Table 1.

### (Measurement of pore volume of each of blood component adsorbent materials 16 to 22)

The pore volume per 1 g of each of the blood component adsorbent materials 16 to 22 was measured in the same manner as the pore volume per 1 g of the blood component adsorbent material 1 was measured. The results are tabulated in Table 2.

### (Measurement of ratio of content of second polymer in blood component adsorbent material 1 as uniformized whole)

The blood component adsorbent material 1 was dried using a warm air drier at 60°C for 4 hours. The weight of the blood component adsorbent material 1 obtained after drying was regarded as the dry weight of blood component adsorbent material 1, and 1 g of the material was weighed out. Furthermore, the material was then impregnated with tetrahydrofuran, 6 N hydrochloric acid, and a 6 N aqueous sodium hydroxide solution at 90°C or more overnight each, then washed with ion-exchanged water five times, and treated in a vacuum-drier for 12 hours. The weight was measured again, the value obtained was regarded as the weight of the blood component adsorbent material 1 after elution, and the value obtained from the formula 10 was rounded to one decimal place to calculate the ratio of content of the second polymer in the blood component adsorbent material 1 as a uniformized whole. The results are tabulated in Table 1. Ratio of content (mg/g) of second polymer in blood component adsorbent material 1 as uniformized whole = {dry weight (1000 mg) - weight after elution (mg)} / 1 g

### (Measurement of ratio of content of second polymer in surface of blood component adsorbent material 1):

On a Nicolet iS5 FT-IR (manufactured by Thermo Scientific; with iD5 diamond ATR accessories; the detector, DTGS KBr; the beam splitter, KBr), the Advanced ATR mode was selected, and the parameters (the number of scans, 16; the data interval, 0.241 cm-1; without automated atmospheric correction) were set. After the setting, a background measurement was made. When the background measurement ended, the blood component adsorbent material 1 preliminarily dried with a warm air drier at 60°C for 4 hours was spread over a prism, and pressed against the prism until the pressure device was locked. A measurement was started within 20 minutes after the background measurement, and then, an infrared absorption spectrum of the surface of the blood component adsorbent material 1 was obtained.

Furthermore, 1 g of the blood component adsorbent material 1 preliminarily dried with a warm air drier at 60°C for 4 hours was weighed out, and was placed in a mortar (an agate mortar manufactured by As One Corporation, deep type, 70 in diameter × 90 in diameter × 30 mm), and ground down 100 times using a pestle with a force that at least cracked the porous particles. The result that all the porous particles were ground down one or more times was verified by visual observation. Except that the resulting powder was used in place of the porous particles, a measurement was made by the same method as the measurement of an infrared absorption spectrum of the surfaces of the porous particles, thus making it possible to obtain an infrared absorption spectrum of the blood component adsorbent material 1 as a uniformized whole. The ratio of content of the second polymer in the surface of the blood component adsorbent material 1 was calculated by rounding, to one decimal place, a value obtained in accordance with the following formula 11 on the basis of the following: the absorption intensity of a peak (2920 cm⁻¹) derived from the CH stretching on each spectrum; and the absorption intensity of a peak (3340 cm⁻¹) derived from the OH group. In this regard, the value of the "ratio of content of the second polymer in the blood component adsorbent material as one uniformized whole" to be used for the formula 11 was calculated using the value calculated in accordance with the formula 10, in which the value is a value not yet rounded to one decimal place. The results are tabulated in Table 1. Ratio of content (mg/g) of second polymer in surface of blood component adsorbent material 1 = ratio of content (mg/g) of second polymer in blood component adsorbent material 1 as uniformized whole × {absorption intensity of peak derived from OH group on surface of blood component adsorbent material 1 / absorption intensity of peak derived from CH stretching on surface of blood component adsorbent material 1} / {absorption intensity of peak derived from OH group in blood component adsorbent material 1 as uniformized whole / absorption intensity of peak derived from CH stretching in blood component adsorbent material 1 as uniformized whole}

### (Measurement of ratio of content of second polymer in blood component adsorbent materials 2 to 15 as uniformized whole)

The ratio of content of the second polymer in each of the blood component adsorbent materials 2 to 15 each as a uniformized whole was measured in the same manner as the ratio of content of the second polymer in the blood component adsorbent material 1 as a uniformized whole was measured. The results are tabulated in Table 1.

### (Measurement of ratio of content of second polymer in surface of each of blood component adsorbent materials 2 to 15)

The ratio of content of the second polymer in the surface of each of the blood component adsorbent materials 2 to 15 was measured in the same manner as the ratio of content of the second polymer in the surface of the blood component adsorbent material 1 was measured. The results are tabulated in Table 1.

### (Measurement of ratio of content of second polymer in each of blood component adsorbent materials 16 to 22 as uniformized whole)

The ratio of content of the second polymer in each of the blood component adsorbent materials 16 to 22 each as a uniformized whole was measured in the same manner as the ratio of content of the second polymer in the blood component adsorbent material 1 as a uniformized whole was measured. The results are tabulated in Table 2.

### (Measurement of ratio of content of second polymer in surface of each of blood component adsorbent materials 16 to 22)

The ratio of content of the second polymer in the surface of each of the blood component adsorbent materials 16 to 22 was measured in the same manner as the ratio of content of the second polymer in the surface of the blood component adsorbent material 1 was measured. The results are tabulated in Table 2.

**[Table 1]**

| Blood Component Adsorbent Material | First Polymer | Second Polymer | Volume of Pores (cm³/g) | Ratio of Content of Second Polymer in Blood Component Adsorbent Material as Uniformized Whole (mg/g) | Ratio of Content of Second Polymer in Surface of Blood Component Adsorbent Material (mg/g) |
|---|---|---|---|---|---|
| Blood Component Adsorbent Material 1 | poly(ethylvinylbenzene/ divinylbenzene) | none | 1.0 | 0 | 0 |
| Blood Component Adsorbent Material 2 | poly(ethylvinylbenzene/ divinylbenzene) | polyvinyl alcohol (ratio of saponification, 10%) | 1.0 | 0.1 | 15 |
| Blood Component Adsorbent Material 3 | poly(ethylvinylbenzene/ divinylbenzene) | polyvinyl alcohol (ratio of saponification, 35%) | 1.0 | 0.2 | 14 |
| Blood Component Adsorbent Material 4 | poly(ethylvinylbenzene/ divinylbenzene) | polyvinyl alcohol (ratio of saponification, 95%) | 1.0 | 0.1 | 14 |
| Blood Component Adsorbent Material 5 | poly(ethylvinylbenzene/ divinylbenzene) | polyvinyl alcohol (ratio of saponification, 95%) | 1.0 | 0 | 3 |
| Blood Component Adsorbent Material 6 | poly(ethylvinylbenzene/ divinylbenzene) | polyvinyl alcohol (ratio of saponification, 95%) | 1.0 | 0.2 | 28 |
| Blood Component Adsorbent Material 7 | poly(ethylvinylbenzene/ divinylbenzene) | polyvinyl alcohol (ratio of saponification, 95%) | 1.0 | 0.3 | 33 |
| Blood Component Adsorbent Material 8 | poly(ethylvinylbenzene/ divinylbenzene) | polyvinyl alcohol (ratio of saponification, 95%) | 1.0 | 0 | 4 |
| Blood Component Adsorbent Material 9 | poly(ethylvinylbenzene/ divinylbenzene) | poly(ethylene/vinyl alcohol) (ratio of ethylene, 40%) | 1.0 | 0 | 5 |
| Blood Component Adsorbent Material 10 | poly(ethylvinylbenzene/ divinylbenzene) | polyvinyl alcohol (ratio of saponification, 95%) | 1.0 | 3.3 | 4 |
| Blood Component Adsorbent Material 11 | poly(ethylvinylbenzene/divinylbenzene) | poly (2-hydroxyethyl methacrylate) | 1.0 | 0.1 | 12 |
| Blood Component Adsorbent Material 12 | poly(ethylvinylbenzene/ divinylbenzene) | dextran sulfate | 1.0 | 0.1 | 8 |
| Blood Component Adsorbent Material 13 | poly(ethylvinylbenzene/ divinylbenzene) | polyvinyl alcohol (ratio of saponification, 95%) | 0.6 | 0.1 | 16 |
| Blood Component Adsorbent Material 14 | poly(ethylvinylbenzene/ divinylbenzene) | polyvinyl alcohol (ratio of saponification, 95%) | 1.5 | 0.1 | 18 |
| Blood Component Adsorbent Material 15 | poly(ethylvinylbenzene/ divinylbenzene) | polyvinyl alcohol (ratio of saponification, 95%) | 1.8 | 0.1 | 17 |

**[Table 2]**

| Blood Component Adsorbent Material | First Polymer | Second Polymer | Volume of Pores (cm³/g) | Ratio of Content of Second Polymer in Blood Component Adsorbent Material as Uniformized Whole (mg/g) | Ratio of Content of Second Polymer in Surface of Blood Component Adsorbent Material (mg/g) |
|---|---|---|---|---|---|
| Blood Component Adsorbent Material 16 | poly(styrene/ ethylvinylbenzene/ divinylbenzene) | polyvinyl alcohol (ratio of saponification, 100%) | 1.2 | 0 | 18 |
| Blood Component Adsorbent Material 17 | poly(ethylvinylbenzene/ divinylbenzene) | poly(4-hydroxymethyl acrylate) | 1.0 | 0 | 22 |
| Blood Component Adsorbent Material 18 | poly(ethylvinylbenzene/ divinylbenzene) | lactose | 1.0 | 0 | 6 |
| Blood Component Adsorbent Material 19 | poly(ethylvinylbenzene/ divinylbenzene) | dextran | 1.0 | 0 | 18 |
| Blood Component Adsorbent Material 20 | poly(styrene/ ethylvinylbenzene/ divinylbenzene) | polyvinyl alcohol (ratio of saponification, 95%) | 1.2 | 0 | 18 |
| Blood Component Adsorbent Material 21 | poly(ethylvinylbenzene/ divinylbenzene/ glycidyl ether ring-opened product) | none | 0.8 | 0 | 0 |
| Blood Component Adsorbent Material 22 | poly(ethylvinylbenzene/ divinylbenzene/ chloromethylstyrene) | polyvinyl alcohol (ratio of saponification, 95%) | 1.0 | 0.1 | 14 |

In Table 1 and Table 2, the "First Polymer" means the name of the first polymer contained in the blood component adsorbent material, and the "second polymer" means the name of the second polymer contained in the blood component adsorbent material.

### (Example 1)

To verify the adsorption capability of the blood component adsorbent material 2, blood was passed through a column packed with the blood component adsorbent material 2 for a predetermined time, a decrease in the amount of leucocytes in the solution between before and after the passing was measured, and the ratio of adsorption of leucocytes was calculated. Below, the measurement method and calculation method of the ratio of adsorption of leucocytes will be described.

A column was produced, which was a cylindrical column (1 cm in inner diameter × 5.14 cm in height) having an inlet and an outlet for blood in the upper and lower portions thereof, and packed with 3.1 mL of a blood component adsorbent material 2. Through this column, human blood having a temperature kept at 37°C (outside temperature) is passed through at a flow rate of 1.9 mL/min for 5 minutes. After the 5 minutes, the blood at the inlet of the column and the blood components at the outlet of the column are analyzed, and the ratio of adsorption of leucocytes by the blood component adsorbent material 2 was calculated. The results are tabulated in Table 1. In this regard, the number of blood components of each kind was measured using a multi-channel automatic blood corpuscle analyzer XT-1800i (manufactured by Sysmex Corporation). The ratio of adsorption of each blood component was calculated in accordance with the following formula 12. The results are tabulated in Table 3. Ratio (%) of adsorption of leucocytes by blood component adsorbent material 2 = {(total number of granulocytes and monocytes in blood at inlet of column) - (total number of granulocytes and monocytes in blood at outlet of column)} / (total number of granulocytes and monocytes in blood at inlet of column) × 100

### (Measurement of ratio of adsorption of IL-8 by blood component adsorbent material 2)

To verify the IL-8 adsorption capability of the blood component adsorbent material 2, the blood component adsorbent material 2 was impregnated with an IL-8-containing liquid for a predetermined time, and then taken out. The ratio of adsorption of IL-8 was measured from a difference in the amount of IL-8 in the liquid between before and after the impregnation. Below, the measurement method will be described.

The blood component adsorbent material 2 in an amount of 0.1 mL was placed in a polypropylene-made container. Into this container, fetal bovine serum (hereinafter referred to as FBS) prepared to have IL-8 at a concentration of 2000 pg/mL was added to make up 30 mL with respect to 1 cm³ of the blood component adsorbent material 2, and mixed by inverting in an incubator at 37°C for 2 hours. Then, the concentration of IL-8 in the FBS was measured by an enzyme-linked immunosorbent assay (ELISA). From the concentration of IL-8 before the inversion mixing, the ratio of adsorption of IL-8 was calculated in accordance with the following formula 13. The results are tabulated in Table 3. Ratio (%) of adsorption of IL-8 by blood component adsorbent material 2 = { concentration (pg/mL) of IL-8 before inversion mixing - concentration (pg/mL) of IL-8 after inversion mixing} / concentration (pg/mL) of IL-8 before inversion mixing × 100

### (Example 2)

Using the blood component adsorbent materials 3 to 6 and 8 to 15, measurements were made in the same manner as in Example 1 to measure the ratio of adsorption of leucocytes and the ratio of adsorption of IL-8. The results are tabulated in Table 3.

### (Comparative Example 1)

Using the blood component adsorbent materials 1 and 7, measurements were made in the same manner as in Example 1 to measure the ratio of adsorption of leucocytes and the ratio of adsorption of IL-8. The results are tabulated in Table 3.

**[Table 3]**

| | Blood Component Adsorbent Material | Ratio of Adsorption of IL-8 (%) | Ratio of Adsorption of Leucocytes (%) |
|---|---|---|---|
| Example 1 | Blood Component Adsorbent Material 2 | 83 | 23 |
| Example 2 | Blood Component Adsorbent Material 3 | 67 | 61 |
| Example 2 | Blood Component Adsorbent Material 4 | 80 | 80 |
| Example 2 | Blood Component Adsorbent Material 5 | 84 | 27 |
| Example 2 | Blood Component Adsorbent Material 6 | 84 | 33 |
| Example 2 | Blood Component Adsorbent Material 8 | 83 | 56 |
| Example 2 | Blood Component Adsorbent Material 9 | 83 | 71 |
| Example 2 | Blood Component Adsorbent Material 10 | 18 | 55 |
| Example 2 | Blood Component Adsorbent Material 11 | 75 | 76 |
| Example 2 | Blood Component Adsorbent Material 12 | 71 | 72 |
| Example 2 | Blood Component Adsorbent Material 13 | 67 | 78 |
| Example 2 | Blood Component Adsorbent Material 14 | 74 | 73 |
| Example 2 | Blood Component Adsorbent Material 15 | 42 | 69 |
| Comparative Example 1 | Blood Component Adsorbent Material 1 | 86 | 3 |
| Comparative Example 1 | Blood Component Adsorbent Material 7 | 73 | 13 |

In Table 3, the "Ratio of Adsorption of Leucocytes" means the ratio of adsorption of leucocytes by the blood component adsorbent material, and the "Ratio of Adsorption of IL-8" means the ratio of adsorption of IL-8 by the blood component adsorbent material.

The results in Table 3 have revealed that a blood component adsorbent material according to the present embodiment adsorbs a blood component such as a leucocyte with high efficiency.

### (Example 3)

Using the blood component adsorbent materials 16, measurements were made in the same manner as in Example 1 to measure the ratio of adsorption of leucocytes and the ratio of adsorption of IL-8. The results are tabulated in Table 5.

### (Example 4)

Using the blood component adsorbent materials 17, measurements were made in the same manner as in Example 1 to measure the ratio of adsorption of leucocytes and the ratio of adsorption of IL-8. The results are tabulated in Table 5.

### (Example 5)

Using the blood component adsorbent materials 19, measurements were made in the same manner as in Example 1 to measure the ratio of adsorption of leucocytes and the ratio of adsorption of IL-8. The results are tabulated in Table 5.

### (Example 6)

Using the blood component adsorbent materials 20, measurements were made in the same manner as in Example 1 to measure the ratio of adsorption of leucocytes and the ratio of adsorption of IL-8. The results are tabulated in Table 5.

### (Example 7)

Using the blood component adsorbent materials 22, measurements were made in the same manner as in Example 1 to measure the ratio of adsorption of leucocytes and the ratio of adsorption of IL-8. The results are tabulated in Table 5.

### (Example 8)

A column was produced, which was a cylindrical column (1 cm in inner diameter × 5.14 cm in height) having an inlet and an outlet for blood in the upper and lower portions thereof, and packed with 3.1 mL of a blood component adsorbent material 4. Through this column, human blood having a temperature kept at 37°C (outside temperature) was passed through at a flow rate of 1.9 mL/min for 5 minutes. After the 5 minutes, the blood at the inlet of the column and the blood at the outlet of the column were sampled, and each of the number of monocytes, the number of neutrophils, the number of basophils, and the number of eosinophils was obtained using a multi-channel automatic blood corpuscle analyzer XT-1800i (manufactured by Sysmex Corporation). The values obtained in accordance with the following formulae 4 to 7 were rounded off to whole numbers, and the ratio of removal of monocytes, the ratio of removal of neutrophils, the ratio of removal of basophils, and the ratio of removal of eosinophils by the blood component adsorbent material 4 were calculated. The results are tabulated in Table 6.

### (Example 9)

The blood component adsorbent material 4 in an amount of 5 mL and distilled water in an amount of 25 mL were placed in a 50 mL centrifuge tube. The resulting mixture was irradiated with a gamma beam at an absorbed dose of 40 kGy to obtain a blood component adsorbent material 4 after sterilization.

A measurement was made by the same procedures as in Example 1 except that the blood component adsorbent material 2 was changed to the blood component adsorbent material 4 after sterilization. The resulting ratio of adsorption of leucocytes was regarded as the ratio of adsorption of leucocytes after sterilization.

A value obtained in accordance with the formula 8 from the ratio of adsorption of leucocytes by the blood component adsorbent material 4 and the ratio of adsorption of leucocytes by the material after sterilization, in which the ratios were obtained in Example 2, was rounded off to whole numbers to calculate the ratio of decrease in the capability of the blood component adsorbent material 4 between before and after the sterilization. The results are tabulated in Table 4.

### (Example 10)

A measurement was made by the same procedures as in Example 9 except that the blood component adsorbent material 4 was changed to the blood component adsorbent material 12. The ratio of adsorption of leucocytes after sterilization was thus obtained. Furthermore, a value obtained in accordance with the formula 8 from the ratio of adsorption of leucocytes by the blood component adsorbent material 12 and the ratio of adsorption of leucocytes by the material after sterilization, in which the ratios were obtained in Example 2, was rounded off to whole numbers to calculate the ratio of decrease in the capability of the blood component adsorbent material 12 between before and after the sterilization. The results are tabulated in Table 4.

### (Example 11)

A measurement was made by the same procedures as in Example 9 except that the blood component adsorbent material 4 was changed to the blood component adsorbent material 17. The ratio of adsorption of leucocytes after sterilization was thus obtained. Furthermore, a value obtained in accordance with the formula 8 from the ratio of adsorption of leucocytes by the blood component adsorbent material 17 and the ratio of adsorption of leucocytes by the material after sterilization, in which the ratios were obtained in Example 4, was rounded off to whole numbers to calculate the ratio of decrease in the capability of the blood component adsorbent material 17 between before and after the sterilization. The results are tabulated in Table 4.

### (Example 12)

A measurement was made by the same procedures as in Example 9 except that the blood component adsorbent material 4 was changed to the blood component adsorbent material 19. The ratio of adsorption of leucocytes after sterilization was thus obtained. Furthermore, a value obtained in accordance with the formula 8 from the ratio of adsorption of leucocytes by the blood component adsorbent material 19 and the ratio of adsorption of leucocytes by the material after sterilization, in which the ratios were obtained in Example 5, was rounded off to whole numbers to calculate the ratio of decrease in the capability of the blood component adsorbent material 19 between before and after the sterilization. The results are tabulated in Table 4.

**[Table 4]**

| | Blood Component Adsorbent Material | Ratio of Adsorption of Leucocytes after Sterilization (%) | Ratio of Decrease in Capability between before and after Sterilization (%) |
|---|---|---|---|
| Example 9 | Blood Component Adsorbent Material 4 | 68 | 15 |
| Example 10 | Blood Component Adsorbent Material 12 | 26 | 65 |
| Example 11 | Blood Component Adsorbent Material 17 | 72 | 13 |
| Example 12 | Blood Component Adsorbent Material 19 | 30 | 55 |

In Table 4, the "Ratio of Adsorption of Leucocytes after Sterilization" means the ratio of adsorption of leucocytes by the blood component adsorbent material after sterilization, and the Ratio of Decrease in Capability between before and after Sterilization means the ratio of decrease in the capability of the blood component adsorbent material between before and after sterilization.

The results in Table 4 have revealed that a blood component adsorbent material according to the present embodiment containing polyvinyl alcohol and poly(4-hydroxybutyl acrylate), which are synthetic polymers, as second polymers has a smaller ratio of decrease in the capability between before and after sterilization than a blood component adsorbent material according to the present embodiment containing dextran sulfate and dextran, which are naturally-occurring polymers, as second polymers.

### (Example 13)

A column was produced, which was a cylindrical column (1 cm in inner diameter × 5.14 cm in height) having an inlet and an outlet for blood in the upper and lower portions thereof, and packed with 3.1 mL of a blood component adsorbent material 4. To human blood kept at 37°C (outside temperature), nafamostat mesilate as an anticoagulant agent was added so as to be at 200 µg/mL, and the resulting mixture was promptly passed through the column at a flow rate of 0.7 mL/min for 5 minutes. After the 5 minutes, the blood at the inlet of the column and the blood at the outlet of the column were sampled, and the number of platelets was obtained using a multi-channel automatic blood corpuscle analyzer XT-1800i (manufactured by Sysmex Corporation). A value obtained in accordance with the formula 9 was rounded off to whole numbers to calculate the ratio of adsorption of platelets by the blood component adsorbent material 4. The results are tabulated in Table 7.

### (Example 14)

A measurement was made by the same procedures as in Example 13 except that the blood component adsorbent material 4 was changed to the blood component adsorbent material 25. The ratio of adsorption of platelets by the blood component adsorbent material 25 was calculated. The results are tabulated in Table 7.

### (Comparative Example 2)

Using the blood component adsorbent materials 18, measurements were made in the same manner as in Example 1 to measure the ratio of adsorption of leucocytes and the ratio of adsorption of IL-8. The results are tabulated in Table 5.

### (Comparative Example 3)

Using the blood component adsorbent materials 21, measurements were made in the same manner as in Example 1 to measure the ratio of adsorption of leucocytes and the ratio of adsorption of IL-8. The results are tabulated in Table 5.

**[Table 5]**

| | Blood Component Adsorbent Material | Ratio of Adsorption of IL-8 (%) | Ratio of Adsorption of Leucocytes (%) |
|---|---|---|---|
| Example 3 | Blood Component Adsorbent Material 16 | 80 | 81 |
| Example 4 | Blood Component Adsorbent Material 17 | 83 | 83 |
| Example 5 | Blood Component Adsorbent Material 19 | 75 | 71 |
| Example 6 | Blood Component Adsorbent Material 20 | 80 | 81 |
| Example 7 | Blood Component Adsorbent Material 22 | 81 | 83 |
| Comparative Example 2 | Blood Component Adsorbent Material 18 | 77 | 16 |
| Comparative Example 3 | Blood Component Adsorbent Material 21 | 34 | 10 |

### (Comparative Example 4)

A measurement was made by the same procedures as in Example 8 except that the blood component adsorbent material 4 was changed to the blood component adsorbent material 18. The ratio of removal of monocytes, the ratio of removal of neutrophils, the ratio of removal of basophils, and the ratio of removal of eosinophils by the blood component adsorbent material 18 were calculated. The results are tabulated in Table 6.

**[Table 6]**

| | Blood Component Adsorbent Material | Ratio of Adsorption of Monocytes (%) | Ratio of Adsorption of Neutrophils (%) | Ratio of Adsorption of Basophils (%) | Ratio of Adsorption of Eosinophils (%) |
|---|---|---|---|---|---|
| Example 8 | Blood Component Adsorbent Material 4 | 83 | 81 | 77 | 76 |
| Comparative Example 4 | Blood Component Adsorbent Material 18 | 17 | 16 | 2 | 11 |

In Table 6, the "Ratio of Adsorption of Monocytes" means the ratio of adsorption of monocytes by the blood component adsorbent material, the "Ratio of Adsorption of Neutrophils" means the ratio of adsorption of neutrophils by the blood component adsorbent material, the "Ratio of Adsorption of Basophils" means the ratio of adsorption of basophils by the blood component adsorbent material, and the "Ratio of Adsorption of Eosinophils" means the ratio of adsorption of eosinophils by the blood component adsorbent material.

The results in Table 6 have revealed that a blood component adsorbent material according to the present embodiment based on a van der Waals force adsorbs any kind of leucocyte with higher efficiency than a blood component adsorbent material based on the specific recognition to a sugar chain.

### (Comparative Example 5)

A measurement was made by the same procedures as in Example 13 except that the blood component adsorbent material 4 was changed to the blood component adsorbent material 1. The ratio of adsorption of platelets by the blood component adsorbent material 1 was calculated. The results are tabulated in Table 7.

**[Table 7]**

| | Blood Component Adsorbent Material | Ratio of Adsorption of Platelets (%) |
|---|---|---|
| Example 13 | Blood Component Adsorbent Material 4 | 18 |
| Example 14 | Blood Component Adsorbent Material 25 | 42 |
| Comparative Example 5 | Blood Component Adsorbent Material 1 | 55 |

In Table 7, the Ratio of Adhesion of Platelets means the ratio of adhesion of platelets to the blood component adsorbent material.

The results in Table 7 have revealed that a blood component adsorbent material according to the present embodiment can inhibit the ratio of adsorption of platelets, and particularly that a blood component adsorbent material containing an amide bond in the linker particularly inhibits the ratio of adsorption of platelets.

### Industrial Applicability

A blood component adsorbent material according to the present invention can adsorb leucocytes and the like with high efficiency, and thus, can be utilized as an adsorptive carrier in a column to be used for the purpose of immunodepression before the treatment or transplantation of an inflammatory disease and for the purpose of inhibiting a side effect of a blood preparation, such as fever or infection.

## Claims

1. A blood component adsorbent material comprising: porous particles composed only of a first polymer having an aromatic hydrocarbon group in a repeating unit; and a second polymer having a hydroxy group in a repeating unit, and fixed to the surfaces of said porous particles, wherein the ratio of content of said second polymer in said surface of said blood component adsorbent material is 3 to 30 mg/g.

2. The blood component adsorbent material according to claim 1, wherein said ratio of content of said second polymer in said blood component adsorbent material as a uniformized whole is 0.3 mg/g or less, and wherein the volume of the pores is 0.5 to 1.8 cm³ per 1 g of the material.

3. The blood component adsorbent material according to claim 1 or 2, wherein said first polymer is polystyrene, polyethylvinylbenzene, polydivinylbenzene, poly(ethylvinylbenzene/divinylbenzene), or poly(styrene/divinylbenzene).

4. The blood component adsorbent material according to any one of claims 1 to 3, wherein said second polymer is a polymer selected from the group consisting of polyhydroxyalkyl acrylate, polyhydroxyalkyl methacrylate, and polyvinyl alcohol.

5. The blood component adsorbent material according to any one of claims 1 to 4, for use in the adsorptive removal of leucocytes.

6. A blood purification column comprising said blood component adsorbent material according to any one of claims 1 to 5.
